(19) Europäisches Patentamt / European Patent Office / Office européen des brevets

(11) **EP 4 252 835 B1**

(12) **EUROPEAN PATENT SPECIFICATION**

(45) Date of publication and mention
of the grant of the patent:
**11.03.2026 Bulletin 2026/11**

(21) Application number: **21898167.8**

(22) Date of filing: **29.11.2021**

(51) International Patent Classification (IPC):
***A61N 5/06*** *(2006.01)*

(52) Cooperative Patent Classification (CPC):
**A61N 5/0622;** A61B 2018/00904; A61N 2005/0626;
A61N 2005/0659

(86) International application number:
**PCT/JP2021/043695**

(87) International publication number:
**WO 2022/114195 (02.06.2022 Gazette 2022/22)**

(54) **PHOTOTHERAPEUTIC APPARATUS**

PHOTOTHERAPEUTISCHES GERÄT

APPAREIL PHOTOTHÉRAPEUTIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.11.2020 JP 2020198632**

(43) Date of publication of application:
**04.10.2023 Bulletin 2023/40**

(73) Proprietor: **Teijin Pharma Limited
Tokyo 100-0013 (JP)**

(72) Inventors:
• **NANJO, Takuya**
**Tokyo 100-0013 (JP)**
• **KAWASE, Yuki**
**Tokyo 100-0013 (JP)**

(74) Representative: **Hoffmann Eitle
Patent- und Rechtsanwälte PartmbB
Arabellastraße 30
81925 München (DE)**

(56) References cited:
WO-A1-2018/017811    WO-A1-2018/221284
WO-A1-2019/094808    WO-A1-2020/004516

## Description

FIELD

The present invention relates to a phototherapy device.

BACKGROUND

Phototherapy devices which irradiate an affected area with light rays such as infrared light rays to relieve pain are known. Since treatment by light irradiation requires repetition of light irradiation for several minutes or more daily several times a week, it is preferable that the patients themselves perform light irradiation at home. However, it is difficult for a patient to press a device against a site which cannot be seen by the patient, such as the back or waist, for performing light irradiation. In connection thereto, there have been reported phototherapy devices which enable a patient to easily irradiate a treatment site with light even if the treatment site is located at a position which cannot be seen by the patient (for example, Patent Literature 1).

WO 2020/004516 illustrates a chair-type phototherapy device in which a barrel tip is aligned with a marking on a body based on images captured by a plurality of cameras. However, it is difficult for the patient to accurately perform alignment while observing the monitor, and if the images obtained by the plurality of cameras are not properly combined, alignment for irradiating the treatment site with light is difficult.

WO 2018/017811 A1 describes an image-guided robotic system for performing precision diagnostic and therapeutic medical procedures, and more specifically, to automated (e.g., robotic) systems and methods for controlled application of energy into or through a body surface to targeted cutaneous and subcutaneous tissue regions for hair and skin treatment procedures, including hair removal. WO 2019/094808 A1 describes a tissue ablation treatment device using an RF ablation component that coordinates the energy delivery, imaging, and navigational control in a seamless and cohesive system.

SUMMARY

The present invention aims to provide a phototherapy device with which a light irradiation region can accurately be aligned with a treatment site. The invention is defined by the appended set of claims.

The phototherapy device according to an embodiment of the present disclosure comprises a barrel, a light source which is installed inside the barrel and which emits light, a plurality of imaging units for imaging a marking arranged on a body, a deviation amount calculation unit for calculating a deviation amount among a plurality of images of the marking included in a plurality of images captured by the plurality of imaging units, an image combining unit for combining the plurality of images of the marking based on the calculated deviation amount, a target position determination unit for determining a position of a target in the combined image corresponding to an irradiation region of light from the light source, and a display unit for overlaying and displaying an image of a target on the combined image of the marking.

In the phototherapy device according to an embodiment of the present disclosure, the deviation amount calculation unit preferably calculates the deviation amount from a center position of the plurality of images of the marking.

In the phototherapy device according to an embodiment of the present disclosure, the plurality of imaging units are preferably arranged behind a barrel tip, which is a portion of the barrel in contact with the body, and outside of the barrel.

In the phototherapy device according to an embodiment of the present disclosure, the marking preferably includes a first marking which is larger than the target, and second markings which are smaller than the first marking.

In the phototherapy device according to an embodiment of the present disclosure, the deviation amount calculation unit preferably calculates a position of the first marking based on a position of the second marking, and calculates the deviation amount.

In the phototherapy device according to an embodiment of the present disclosure, it is preferable that there further be provided a target deviation amount calculation unit for calculating a target deviation amount between the target position and a position of the marking.

In the phototherapy device according to an embodiment of the present disclosure, it is preferable that there further be provided an irradiation position judgment unit for judging whether or not the position of the irradiation region is a predetermined irradiation region position based on whether or not the position of the combined image of the marking and the position of the image of the target are aligned.

In the phototherapy device according to an embodiment of the present disclosure, the irradiation position judgment unit preferably judges whether or not the position of the center of the irradiation region of the light from the light source and the position of the center of the target are aligned based on the deviation amount of the target.

In the phototherapy device according to an embodiment of the present disclosure, it is preferable that there further be provided a filter which is installed in front of the plurality of imaging units and which filters emitted light of the light source.

In the phototherapy device according to an embodiment of the present disclosure, the wavelength of the emitted light of the light source preferably does not overlap with an absorption wavelength of the marking.

According to the phototherapy device according to an embodiment of the present disclosure, the light irradiation region can accurately be aligned with the treatment site.

BRIEF DESCRIPTION OF DRAWINGS

FIG. 1 is a configuration view of the phototherapy device according to an embodiment of the present disclosure.

FIG. 2 is a schematic view detailing an aspect of treatment by the phototherapy device according to an embodiment of the present disclosure.

FIG. 3 is a block diagram of the phototherapy device according to an embodiment of the present disclosure.

FIG. 4 is a plane view of a marking used in the phototherapy device according to an embodiment of the present disclosure.

FIG. 5 is a flowchart detailing the procedure for combining images by the phototherapy device according to an embodiment of the present disclosure.

FIG. 6 is a view showing images captured by left and right imaging units in the phototherapy device according to the embodiment of the present disclosure, showing an example in which a marking deviation amount is equal to or less than the width of the screen.

FIG. 7 is a view detailing the procedure for generating a combined image from the two images shown in FIG. 6.

FIG. 8 is a view showing images captured by left and right imaging units in the phototherapy device according to the embodiment of the present disclosure, showing an example in which a marking deviation amount is larger than the width of the screen.

FIG. 9 is a view detailing the procedure for generating a combined image from the two images shown in FIG. 8.

FIG. 10 is a view detailing the procedure for generating a combined image from two images captured by left and right imaging units when a part of the marking overlaps the barrel in the phototherapy device according to the embodiment of the present disclosure.

FIG. 11A shows an example of an image when the target image is displayed on the combined image shown in FIG. 10.

FIG. 11B shows an example of the image when the target position is aligned with the combined marking.

FIG. 12 is a graph showing the relationship between the wavelength of light used in the phototherapy device according to an embodiment of the present disclosure and the absorbance of the marking and skin.

DESCRIPTION OF EMBODIMENTS

The phototherapy device according to the present invention will be described below with reference to the drawings. However, it should be noted that the technical scope of the present invention is not limited to the following embodiments, but extends to the invention described in the claims and equivalents thereof.

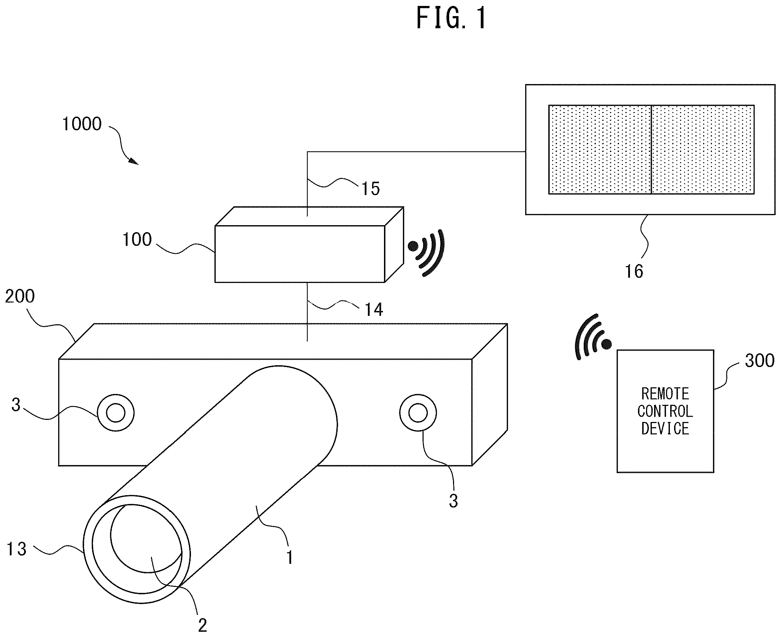

First, the configuration of the phototherapy device according to an embodiment of the present disclosure will be described. FIG. 1 shows a configuration view of a phototherapy device 1000 according to an embodiment of the present disclosure. The phototherapy device 1000 comprises a controller 100 and a light-emitting unit 200. The light-emitting unit 200 irradiates the affected area of the patient with light. The controller 100 is connected to the light-emitting unit 200 by first wiring 14 and controls the light-emitting unit 200. The controller 100 may be wirelessly connected to the light-emitting unit 200.

The light-emitting unit 200 comprises a barrel 1, a light source 2, and an imaging unit 3. The barrel 1 is provided in substantially the center of the light-emitting unit 200 so as to protrude toward the patient. The barrel 1 is a member for aligning the barrel tip 13 with a predetermined position on the skin of the patient and irradiating it with light rays, and is composed of a hollow cylindrical material such as metal or resin. Though FIG. 1 shows an example in which the shape of the barrel 1 is cylindrical, the shape is not limited to cylindrical, and other shapes such as a prism-like shape may be adopted.

The light source 2 is installed inside the barrel 1 and emits light. As the light source 2, for example, a laser, LED, halogen lamp, or xenon lamp can be used. The light source 2 is arranged so that the irradiation light passes along the central axis of the cylindrical barrel 1 and is emitted onto the treatment site of the body. Alternatively, the light source 2 may be installed outside the barrel 1 and light may be guided to the barrel 1 using a light guide path such as an optical fiber. The light emitted from the light source 2 may be infrared light with a wavelength of 600 to 12000 [nm], and in particular, near infrared light having a high skin permeability of 780 to 2500 [nm] can be used. However, as will be described later, it is preferable to set the wavelength of the laser light to 800 [nm] in order to avoid absorption of the laser light by the marking.

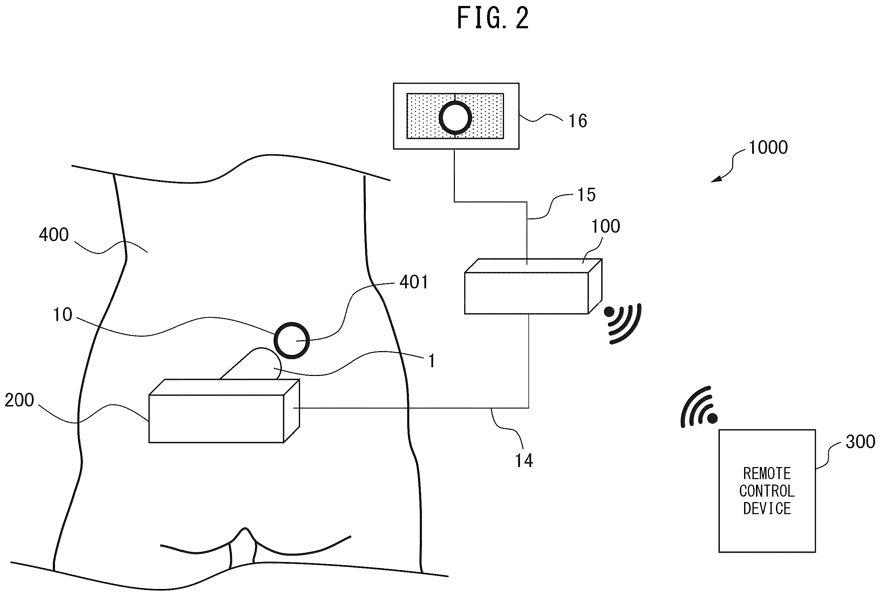

The imaging unit 3 images a marking 10 (refer to FIG. 2) placed on the body of the patient. A plurality of imaging units 3 are preferably provided. By providing a plurality of imaging units 3 and imaging the entire circumference of the barrel 1 with the imaging units 3, alignment of the barrel 1 and the marking can be more accurately performed. The example shown in FIG. 1 shows an example in which two imaging units 3 are provided in the light-emitting unit 200. However, the present invention is not limited to such an example, and three or more imaging units 3 may be provided. The imaging unit 3 makes it possible to recognize the treatment site of the patient and the irradiation position of the irradiation light from the light source 2. A CCD camera or a CMOS camera can be used as the imaging unit 3. Images captured by the imaging unit 3 can be displayed on a display unit 16. The display unit 16 is connected to the light-emitting unit 200 by second wiring 15. Alternatively, the display unit 16 and the light-emitting unit 200 may be wirelessly connected.

The imaging units 3 are preferably provided behind the barrel tip 13 and outside the barrel 1. In FIG. 1, the imaging units 3 are provided coplanar with the surface of the light-emitting unit 200 to which the barrel 1 is connected and outside the barrel 1. The imaging units 3 are attached so as to image the periphery of the barrel 1 or barrel tip 13. The imaging units 3 may be provided on a plane different from the surface of the light-emitting unit 200 to which the barrel 1 is connected, and may be arranged so as to protrude from an arbitrary surface of the light-emitting unit 200.

By providing the imaging units 3 behind the barrel tip 13 and outside the barrel 1, the imaging units 3 do not contact the body of the patient when the barrel tip 13 does not contact the body. Furthermore, by emitting light from the light source 2 only when the barrel tip 13 is in contact with the body, leakage of the irradiation light emitted from the light source 2 and the reflected light thereof out of the barrel 1 can be prevented. Thus, reflected light from the irradiation field does not enter the imaging units 3 installed outside the barrel 1. Therefore, halation caused by the reflected light from the irradiation field in the imaging units 3 can be suppressed.

The phototherapy device 1000 according to the embodiment of the present disclosure may comprise an irradiation control remote control device 300 with which the patient can control ON/OFF of light irradiation. The irradiation control remote control device 300 wirelessly communicates with the controller 100. The irradiation control remote control device 300 transmits a signal to the controller 100 based on the contents input by the patient by operating the switch of the irradiation control remote control device 300. The controller 100 controls light irradiation from the light source 2 of the light-emitting unit 200 based on the received signal. As a result, the patient can turn ON/OFF the light irradiation, whereby erroneous irradiation to areas other than the treatment site is prevented, and phototherapy can safely be performed.

Next, the specific method for using the phototherapy device according to the embodiment of the present disclosure will be described. FIG. 2 shows a schematic view detailing an aspect of treatment by the phototherapy device 1000 according to an embodiment of the present disclosure. First, in a hospital or clinic, the treatment site where the patient 400 should be irradiated with light is identified based on a diagnosis of a physician. For example, in the treatment of dysuria by light irradiation, in order to suppress abnormal activity of the sensory nerves of the bladder, the sacral foramen where the sensory nerves of the bladder are present is targeted and irradiated with light percutaneously. Since proper treatment requires accurate targeting of the sacral foramina, the position of the sacral foramina which should be irradiated with light is identified on the back of the patient 400, for example, by palpation or X-ray fluoroscopy.

Next, the marking 10 is then placed by a physician or another medical personnel at the identified treatment site. As the material of the marking 10, ink or an adhesive sheet can be used. For example, in the treatment of dysuria by light irradiation, since it is preferable to repeat light irradiation for several minutes to several tens of minutes daily at a frequency of twice a week to every day, it is desirable that the patient 400 themselves perform light irradiation at home. Therefore, a physician identifies the sacral foramen at a hospital, places a marking 10 indicating the irradiation position and irradiation range on the skin directly thereabove, and the patient 400 at home arranges the barrel 1 of the phototherapy device 1000 at an appropriate position according to the marking 10, and performs light irradiation.

The patient 400 uses the phototherapy device 1000 to irradiate themselves with light at home. The patient 400 affixes the light-emitting unit 200 and aligns the position of the marking 10 applied to the treatment site with the position of the barrel 1 while adjusting the position of their own body. When the treatment site is on the back of the patient 400 and cannot be seen by themself, the patient 400 adjusts the position of their own body so that the position of the marking 10 is aligned with the position of the barrel 1 while viewing the images captured by the imaging units 3 (refer to FIG. 1) on the display unit 16. For example, the position of the body is adjusted so that the barrel tip 13 fits inside the hollow circular shape of the marking 10. Thereafter, light irradiation is performed by operating the irradiation control remote control device 300.

Since accurate alignment becomes difficult if the marking 10 is hidden by the barrel tip 13 when the barrel tip 13 contacts the skin of the patient 400, the marking 10 is preferably shaped so as to avoid the barrel tip 13. For example, marking 10 may have a hollow circular shape having a radius greater than the radius of barrel tip 13. However, the shape of the marking is not limited to such an example, and the shape of the marking may be another shape in accordance with the shape of the target corresponding to the irradiation region of the light from the light source 2.

The phototherapy device according to an embodiment of the present disclosure is a phototherapy device which achieves accurate alignment by aligning the marking on the body with a target displayed on the display unit 16. By using the phototherapy device according to the embodiment of the present disclosure, effective and simple treatment for the patient can be realized.

The images captured by the two or more cameras are combined and displayed on the display unit 16. When combining, the deviation amounts of the images in each direction are calculated based on the size and position of the marking on the body, and the images are superimposed (stitched) according to the values to display a highly visible combined image. The indicator serving as a target for alignment is superimposed and displayed on the display unit 16 in accordance with the position of the light irradiation field within the barrel. As a result, the actual light irradiation position and the marking on the body can accurately be aligned.

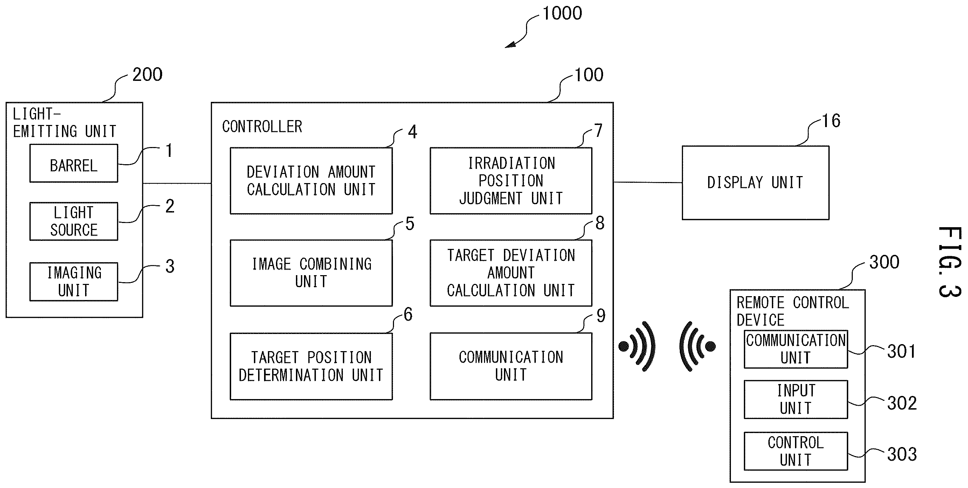

Next, the configuration of the phototherapy device according to an embodiment of the present disclosure will be described in detail. FIG. 3 shows a block diagram of the phototherapy device 1000 according to an embodiment of the present disclosure. The phototherapy device 1000 comprises the controller 100 and the light-emitting unit 200. The configuration of the light-emitting unit 200 is as described above.

The controller 100 comprises a deviation amount calculation unit 4, an image combining unit 5, a target position determination unit 6, and the display unit 16. The controller 100 preferably further comprises an irradiation position judgment unit 7, a target deviation amount calculation unit 8, and a communication unit 9. The deviation amount calculation unit 4, image combining unit 5, target position determination unit 6, irradiation position judgment unit 7, and target deviation amount calculation unit 8 can be realized by executing programs stored in a storage unit (not illustrated) by a processor such as a CPU provided in the controller 100. The storage unit may be a computer-readable recording medium. A liquid crystal display device or an organic EL display device can be used as the display unit 16.

The phototherapy device 1000 preferably further comprises an irradiation control remote control device 300. The irradiation control remote control device 300 comprises a communication unit 301, an input unit 302, and a control unit 303. The communication unit 301 wirelessly communicates with the communication unit 9 of the controller 100 to transmit and receive data for controlling light irradiation. The irradiation control remote control device 300 may be connected via wire to the controller 100. The input unit 302 is used by the patient to perform the operation for turning ON/OFF light irradiation. The control unit 303 controls the communication unit 301 and the input unit 302. The control unit 303 comprises a CPU, and controls the irradiation control remote control device 300 by executing programs stored in the storage unit (not illustrated).

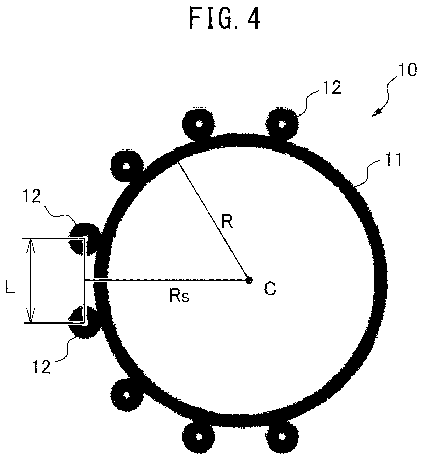

FIG. 4 shows a plane view of the marking 10 used in phototherapy device 1000 according to an embodiment of the present disclosure. The marking 10 is an indicator for performing light irradiation on a desired affected region by calculating the position of the center and aligning it with the center of the target. The marking 10 preferably comprises a first marking 11 which is larger than the target and second markings 12 which are smaller than the first marking 11. The target is a region which is inside the barrel tip 13 and which is irradiated with light from the light source 2. The first marking 11 preferably has a radius which is greater than the radius of the barrel tip 13. Thus, the first marking 11 is preferably larger than the target.

Though FIG. 4 shows an example in which both the first marking 11 and the second markings 12 forming the marking 10 are circular, the present invention is not limited to such an example. For example, the first marking 11 and the second markings 12 may be oval-shaped, rectangular, square, or polygonal. The first marking 11 and the second markings 12 may not have similar shapes. For example, the first marking 11 may be circular and the second markings 12 may be square.

The shape of the first marking 11 is preferably similar to the shape of the target and larger than the barrel tip 13. By making the shape of the first marking 11 larger than the shape of the barrel tip 13, the position of the light irradiation can easily be aligned with the position of the first marking 11.

Though second markings 12 are arranged at a part of the vertices of a regular dodecagon on the circle of the first marking 11 in the example shown in FIG. 4, the present invention is not limited to such an example. For example, the positions of the second markings 12 can be arranged on at least a part of the vertices of any regular polygon on the circle of the first marking 11.

Furthermore, the second markings 12 need not be arranged at all the vertices of the regular polygon of the first marking 11, and may be arranged at some of the vertices. In the example shown in FIG. 4, among the 12 vertices of a regular dodecagon, markings 12 are arranged on the 8 vertices on the left side. The second markings 12 are arranged in such a manner on part of the first marking 11 because by arranging a plurality of second markings, the positions of markings thereamong to be hidden by the barrel 1 have been revealed in advance. For example, in the example shown in FIG. 4, when an imaging unit 3 is arranged on the left side of barrel 1, the barrel 1 is reflected on the right side of the marking 10, and a part of the right side of the marking 10 overlaps with the barrel 1 and becomes invisible. Thus, when an additional marking is arranged on the right side of the marking 10 shown in FIG. 4, at the time of observation with the imaging unit 3 arranged on the right side of the barrel 1, since the barrel 1 obscures the left side of the additional marking, second markings need be placed to only the right of the first marking of the additional marking.

When the entirety of the first marking 11 is visible without overlapping the barrel 1, the center position can be calculated from the first marking 11 alone. However, when the barrel 1 is brought closer to the first marking 11, a part of the first marking 11 overlaps with the barrel 1, such that the part of first marking 11 becomes invisible. In such cases, the center position cannot be calculated from the first marking 11 alone. Thus, the position of the center of the first marking 11 is calculated using the second markings 12 arranged around the first marking 11. For example, as shown in FIG. 4, a plurality of second markings 12 are arranged at the vertices of a regular dodecagon. Since the positional relationship between the second markings 12 arranged at the vertices of the regular dodecagon and the first marking 11 is known, if two adjacent second markings 12 can be detected, the position of the center C of the first marking 11 can be calculated. Further, the radius of the first marking 11 can also be calculated from the positions of two adjacent second markings 12. The method for calculating the radius of the first marking 11 from the positions of the second markings 12 is described below.

R is the radius of the first marking 11, Rs is the distance from the center C to the line connecting the centers of adjacent second markings 12, and L is the distance between the centers of the adjacent second markings 12. At this time, L can actually be measured from the center positions of the two adjacent second markings 12. When the radius R is set to 21 mm and Rs is premeasured as 23.3 mm, since the angle formed by the two lines connecting the center C of the first marking 11 and the centers of the adjacent second markings 12 is 30 degrees, the radius R can be calculated from the measured value L using the following formula (1).
[Math 1]

$$R = \frac{21.0}{23.3} \cdot \frac{L}{2 \tan(15°)} \qquad (1)$$

Formula (1) is based on an example in which the second markings 12 are arranged at the vertices of a regular dodecagon on the circle of the first marking 11. However, the formula for calculating the radius of the first marking 11 from the positions of the second markings 12 is not limited to formula (1) above. For example, even when second markings 12 are arranged at the vertices of a regular polygon other than a regular dodecagon on the circle of the first marking 11, the radius of the first marking 11 can be calculated from the positions of the second markings 12.

The deviation amount calculation unit 4 calculates a deviation amount between a plurality of images of the marking included in a plurality of images captured by the plurality of imaging units 3. A function HoughCircles( ) for detecting circles using Hough transformation, which is prepared in OpenCV, can be used in the detection of the marking. By using the HoughCircles( ) function, the position of the center of the first marking 11 can be obtained.

When the first marking 11 approaches the imaging unit 3, the circle is only partially captured and is no longer detected as a circle. In that case, the parameters of the HoughCircles( ) function are changed so as to detect the second markings 12.

The method for calculating the deviation amount between the plurality of images of the marking differs depending on whether or not the first marking 11 can be detected in the plurality of images (for example, the images from the left and right imaging units) captured by the plurality of imaging units 3. First, when the first marking 11 can be detected in both the left and right images captured by the left and right imaging units 3, the deviation amount between the images of the marking in the left and right images is obtained as the difference between the center position of the first marking 11 obtained in each of the left and right images. Second, if the first marking 11 can be detected only in either of the left or right image, or if only the second markings 12 can be detected in both images, since the deviation amount between the images of the marking is proportional to the radius of the first marking 11, the proportional coefficient can be obtained in advance and the deviation amount can be calculated from the radius of the first marking 11.

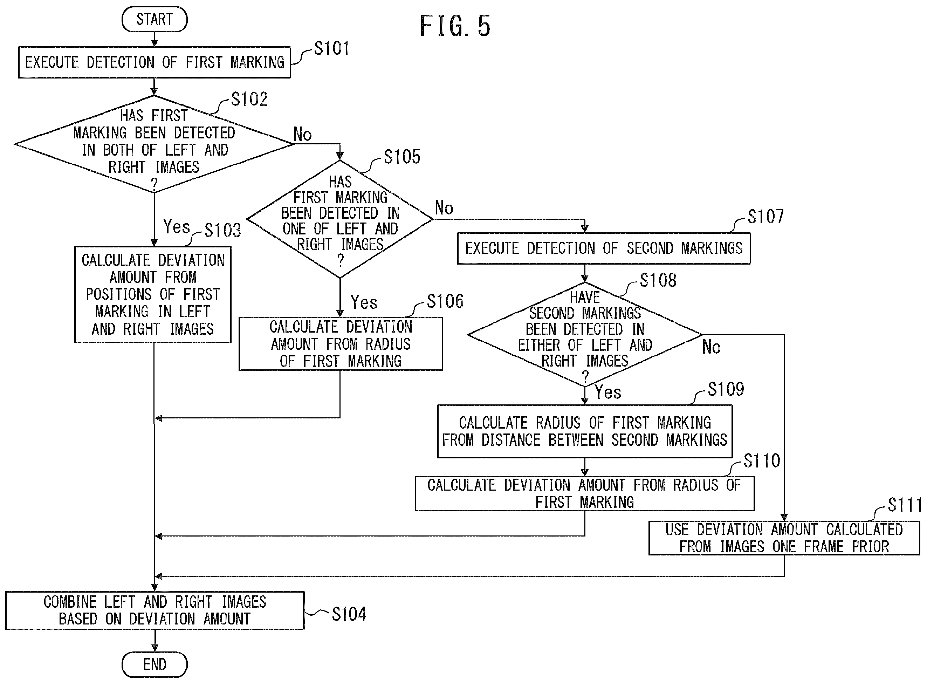

The image combining unit 5 combines the plurality of images of the marking based on the calculated deviation amount. Next, the procedure for combining the images using the detected marking will be described. FIG. 5 shows a flowchart detailing the procedure for combining the images by the phototherapy device according to an embodiment of the present disclosure.

First, in step S101, detection of the first marking 11 is executed from both the left and right images captured by the imaging units 3 provided on the left and right. Next, in step S102, it is determined whether or not the first marking 11 has been detected in both the left and right images. If the first marking 11 has been detected in both the left and right images, in step S103, the deviation amount is calculated from the position of the first marking 11 in the left and right images. Next, in step S104, the left and right images are combined based on the calculated deviation amount. The specific image combination method will be described later.

If the first marking 11 was not detected in both the left and right images in step S102, it is determined in step S105 whether the first marking 11 has been detected in one of the left and right images. If the first marking 11 has been detected in one of the images, in step S106, the deviation amount is calculated from the radius of the first marking 11 in the one image. Next, in step S104, the left and right images are combined based on the calculated deviation amount.

If the first marking 11 has not been detected in either of the left and right images in step S105, detection of the second markings 12 is executed in step S107. Next, in step S108, it is determined whether or not the second markings 12 have been detected in either the left or right image. If the second markings 12 have been detected in either the left or right image, in step S109, the radius R of the first marking 11 is calculated from the distance L between the second markings 12 in accordance with formula (1) above. Next, in step S110, the deviation amount is calculated from the radius of the first marking 11 in one image. Next, in step S104, the left and right images are combined based on the calculated deviation amount.

In step S108, if the second markings 12 have not been detected in either the left or right image, in step S111, the deviation amount obtained from images one frame prior is used. Next, in step S104, the left and right images are combined based on the deviation amount.

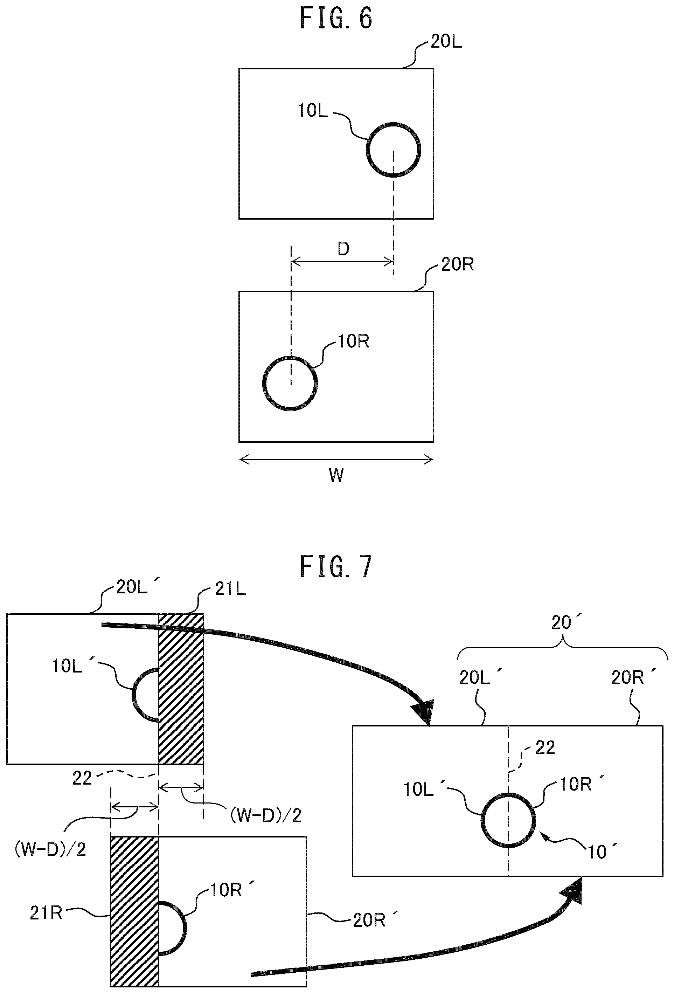

Next, a method of combining two images based on the deviation amount between the two images including the marking will be described. Two image combination methods can be conceived of depending on the relationship between the width of the images and the deviation amount of the marking. The first method corresponds to the case in which the marking deviation amount is less than the width of the screen. FIG. 6 shows images captured by the left and right imaging units 3 in the phototherapy device according to an embodiment of the present disclosure, and shows an example in which the marking deviation amount is equal to or less than the width of the screen. In FIG. 6, marking 10L is displayed on the left image 20L captured by the imaging unit 3 on the left, and marking 10R is displayed on the right image 20R captured by the imaging unit 3 on the right. D is the deviation amount between the markings 10L and 10R, and W is the screen size. In the example shown in FIG. 6, the deviation amount D is equal to or less than the screen width W ($D \leq W$). Note that although the description of the second markings in FIGS. 6 to 9 is omitted in order facilitate understanding, second markings are arranged on at least a part of the vertices of an arbitrary regular polygon arranged on the circle of the markings (10R, 10L, etc.) in the same manner as in FIG. 4.

FIG. 7 shows a view detailing the procedure for generating a combined image from the two images shown in FIG. 6. When the deviation amount between the left and right markings 10L and 10R is known, the hatched regions (21L, 21R) bounded by the dashed line 22 are excluded from the left and right images (20L, 20R). For example, the regions 21L and 21R to be removed can be images of width (W - D)/2. The 10L', which is the remainder of 10L excluding the shaded portion, and the 10R', which is the remainder of 10R excluding the shaded portion, are displayed in the images (20L', 20R'), which are the remainders excluding the shaded regions (21L, 21R).

Next, a combined image 20' is created by arranging and combining the images (20L', 20R'), which are the remainders of the left and right images (20L, 20R) excluding the shaded regions (21L, 21R). At this time, the markings 10R' and 10L', which are the remainders excluding the portions, are combined to generate marking 10' in the combined image 20'.

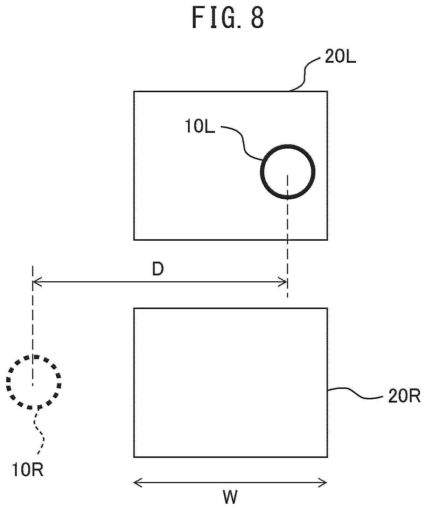

Next, as a second method, the case in which the marking deviation amount is greater than the width of the screen will be described. FIG. 8 shows an example of images captured by the left and right imaging units in the phototherapy device according to an embodiment of the present disclosure when the marking deviation amount is greater than the width of the screen. In FIG. 8, marking 10L is displayed on the left image 20L captured by the imaging unit 3 on the left. Marking 10R is not displayed on the right image 20R captured by the right imaging unit 3, and is present outside the screen 20R, as indicated by the dotted line of FIG. 8. When the deviation amount between the markings 10L and 10R is D and the screen size is W, the deviation amount D is greater than the screen width W (D > W) in the example shown in FIG. 8.

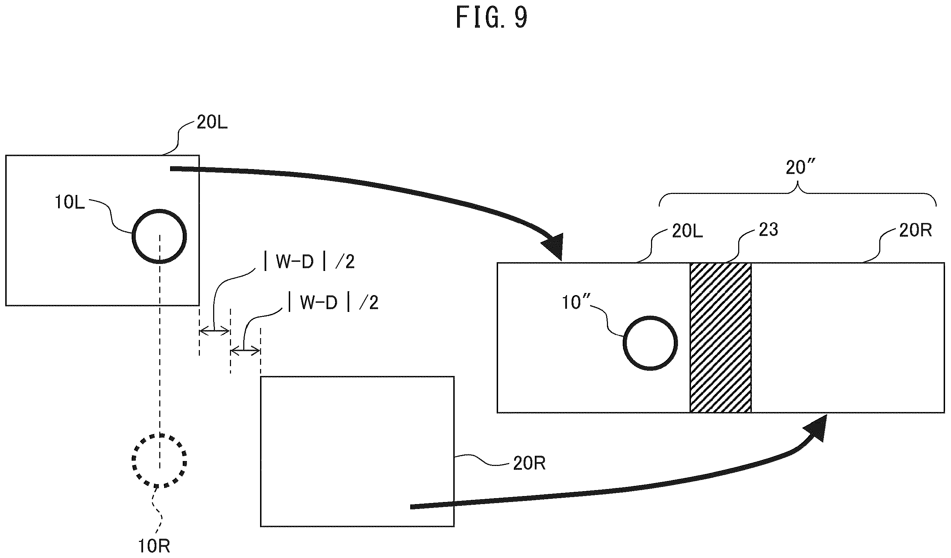

FIG. 9 shows a view detailing the procedure for generating a combined image from the two images shown in FIG. 8. If the deviation amount D between the left and right markings 10L and 10R is greater than the width W of the image, the width of the region to be excluded is negative. Specifically, since the width of the image required for combining is larger than the width of the captured images, the center of the combined image will be a region 23 where no image is present.

Next, an image 23 is added between the images 20L and 20R to generate a combined image 20''. At this time, the combined image 20'' is combined with the markings 10L and 10R to generate marking 10".

By combining the left and right images as described above, a combined marking (10', 10") is generated, the light irradiation region is aligned with the generated marking (10', 10"), and irradiation is carried out.

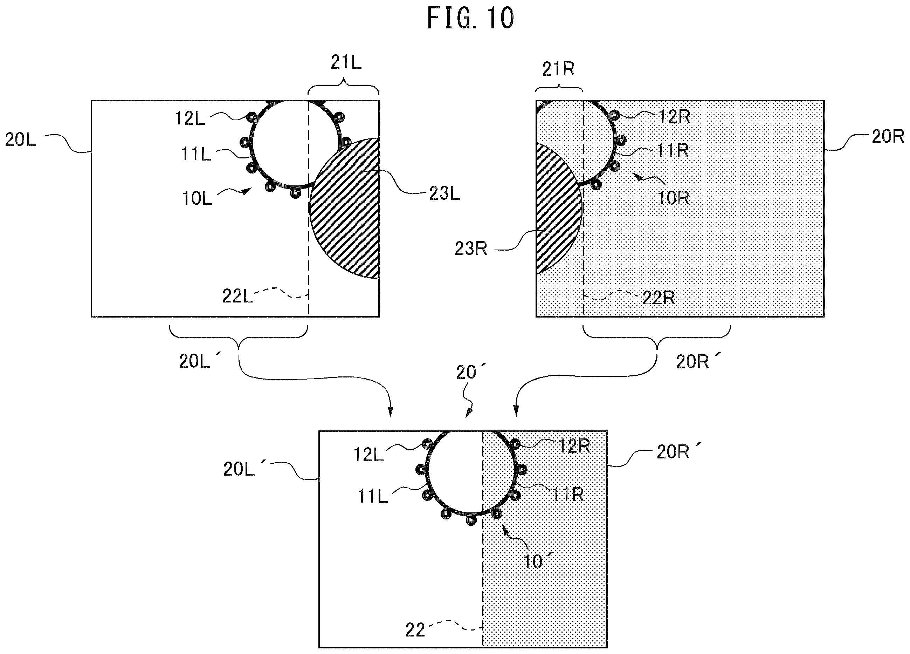

Next, the procedure for combining the markings captured by the two imaging units and superimposing the target representing the position of the light irradiation region on the combined markings will be described. FIG. 10 shows a view detailing the procedure for generating a combined image from two images captured by left and right imaging units when a part of the markings overlaps the barrel in the phototherapy device according to an embodiment of the present disclosure. The left image 20L captured by the left imaging unit 3 and the right image 20R captured by the right imaging unit 3 are shown in the upper part of FIG. 10.

The marking 10L is displayed in the left image 20L. The marking 10L includes a first marking 11L and second markings 12L. An image 23L of a part of the left side of the barrel 1 is reflected in the left image 20L, whereby a part of the first marking 11L is invisible. In this case, the second markings 12L are used to calculate the center position or radius of the first marking 11L.

The marking 10R is displayed in the right image 20R. The marking 10R includes a first marking 11R and second markings 12R. An image 23R of a part of the right side of the barrel 1 is reflected in the right image 20R, whereby a part of the first marking 11R is invisible. In this case, the second markings 12R are used to calculate the center position or radius of the first marking 11R. The deviation amount is then calculated from the calculated center positions or radii of the first markings 11R and 11L.

Next, from the calculated deviation amount, the region 21L on the right side of the dashed line 22L in the left image 20L is excluded to generate the remaining image 20L'. It is preferable to determine the position of the dashed line 22L so that the region 21L to be excluded includes the image 23L of the part of the barrel 1 and the image 23L of the part of the barrel 1 is not included in the remaining image 20L'.

Likewise, the region 21R on the left side of the dashed line 22R in the right image 20R is excluded from the calculated deviation amount to generate the remaining image 20R'. It is preferable to determine the position of the dashed line 22R so that the region 21R to be excluded includes the image 23R of the part of the barrel 1 and the image 23R of the part of the barrel 1 is not included in the remaining image 20R'.

Next, as shown in the lower part of FIG. 10, the remaining images 20L' and 20R' are combined to generate a combined image 20'. The marking 10', in which the markings 10L and 10R are combined, is displayed in the combined image 20'. The dashed lines 22L and 22R overlap at the position of the dashed line 22 in the combined image 20'.

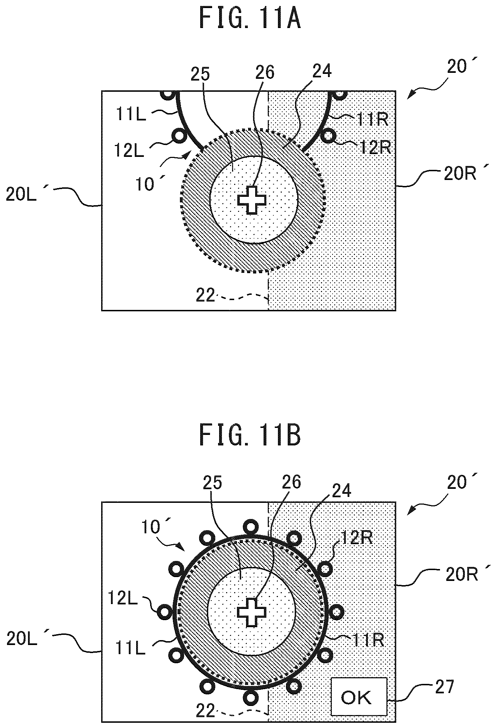

Since the position of the barrel is not displayed in the combined image 20' shown in the lower part of FIG. 10, the marking 10' cannot be aligned with the light irradiation region. Thus, the target is superimposed and displayed on the combined image 20' at a position corresponding to the light irradiation region. FIG. 11A shows an example of a screen when the image of the target 24 is displayed on the combined image 20' shown in FIG. 10. The target position determination unit 6 determines the position of the target 24 corresponding to the irradiation region of the light from the light source 2 on the combined image 20'. For example, the target 24 may be displayed approximately in the center of the combined image 20'. However, the present invention is not limited to such an example. As shown in FIG. 11A, the display unit 16 displays the image of the target 24 superimposed on the combined image of the marking 10'. In FIG. 11A, the barrel tip 13 is brought nearer to the treatment site of the patient than in the step of creating the marking 10' combined from the two images as shown in FIG. 10. Thus, in FIG. 11A, the imaging units 3 are closer to the treatment site than in FIG. 10, whereby the size of the marking 10' displayed in the combined image 20' in FIG. 11A is displayed larger than in the case shown in FIG. 10.

A light irradiation region 25 and a marker 26 indicating the central position of the irradiation region are preferably overlaid and displayed on the target 24. The target deviation amount calculation unit 8 calculates the target deviation amount between the position of the target 24 and the position of the marking 10'. At this time, since a part of the first markings (11R, 11L) constituting the marking 10' overlaps the target 24 and the whole is not displayed, the second markings (12R, 12L) are preferably used to calculate the position of the center of the marking 10'. The target deviation amount can be calculated by the difference between the calculated center position of the marking 10' and the center position of the marker 26.

The irradiation position judgment unit 7 judges whether or not the position of the light irradiation region 25 is the position of the predetermined irradiation region based on whether the position of the image of the combined marking 10' and the position of the image of the target 24 are aligned. The irradiation position judgment unit 7 preferably judges whether or not the center position of the light irradiation region 25 from the light source 2 and the center position of the target 24 are aligned. FIG. 11B shows an example of an image when the target 24 is aligned with the combined marking 10'. Since the actual light irradiation position is present within the barrel 1 at this time, it cannot be captured from the outside imaging units 3. Thus, in the alignment of the marking 10' and the barrel 1, it is unclear whether the center of the marking 10' is aligned with the center of the irradiation beam. However, if the center positions of the target 24 and the irradiation beam are aligned, the beam can be accurately adjusted to a predetermined irradiation position by aligning the target 24 and the marking 10'.

After the alignment between the target 24 and the marking 10' is completed, the irradiation control remote control device 300 is operated to execute light irradiation. When the alignment between the target 24 and the marking 10' is completed, a part of the combined image 20' may be displayed to indicate that the alignment has been completed and that light irradiation may be performed. For example, as shown in FIG. 11B, a display 27 of "OK" may be displayed on a part of the combined image 20'. The patient can confirm that "OK" is displayed on the combined image 20' and recognize that light irradiation can be performed. Alternatively, when alignment between the target 24 and the marking 10' is completed, the patient may be notified by sound from the irradiation control remote control device 300, the display unit 16, or the like that the alignment of a part of the combined image 20' is completed and light irradiation may be performed.

Conversely, if the alignment between the target 24 and the marking 10' is not completed, an indication that alignment has not been completed and light irradiation cannot be performed may be displayed on a part of the combined image 20'. For example, "NG" may be displayed on a part of the combined image 20'. When "NG" is displayed on part of the combined image 20', it is preferable to control so that light irradiation is not executed. This is because the patient is not ready to appropriately irradiate the affected area with light, and the desired effect of the light irradiation cannot be obtained in a displaced state.

Further, when the target 24 and the marking 10' have not been aligned with each other, and the patient operates the irradiation control remote control device 300 to execute light irradiation, it is preferable to warn the patient with a warning sound or the like to make the patient aware that alignment has not been completed. Furthermore, since the irradiation time of light irradiation ranges from several minutes to several tens of minutes, if the positions of the target 24 and the marking 10' are displaced during light irradiation, light irradiation may be stopped at the point of displacement.

As described above, the patient can accurately irradiate the affected area with light by aligning the combined marking 10' with the target 24.

Though an example of alignment using only one marking (first marking) was described in the above description, a plurality of markings (first markings) may be arranged. For example, when there are a plurality of locations to be irradiated, such as when irradiating the nerves running on the left and right sides of the spine one by one, the number of markings may be adjusted accordingly.

If a gap occurs between the barrel tip 13 and the skin of the patient when irradiating the affected area with light, it is conceivable that light will leak out of the barrel 1 and the leaked light enters the imaging unit 3, making the image displayed on the display unit 16 difficult to see. Thus, it is preferable that there further be provided a filter (not illustrated) installed in front of the plurality of imaging units 3 to block light emitted from the light source 2. By providing a filter, even if light leaking from the barrel 1 reaches the imaging units 3, influence thereof on the image displayed on the display unit 16 can be suppressed.

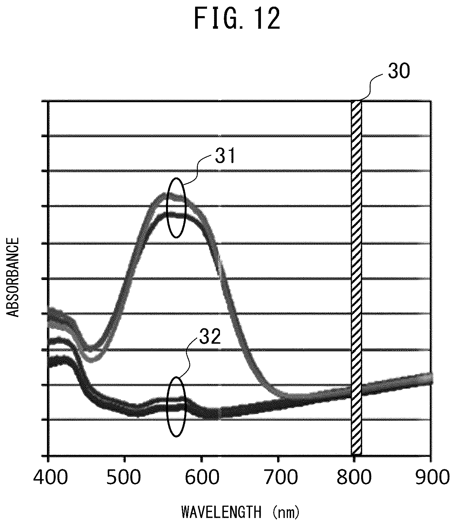

As described above, when a gap occurs between the barrel tip 13 and the skin of the patient, light leaks from the barrel 1 and a part of the leaked light may enter the marking 10, and thus, it is preferable that the wavelength of the emitted light from the light source 2 not overlap the absorption wavelength of the marking 10. This is because when the light from the light source 2 is absorbed by the marking 10, the temperature of the skin on which the marking 10 is arranged rises, which is undesirable. FIG. 12 shows a graph of the wavelength of light used in the phototherapy device according to an embodiment of the present disclosure versus the absorbance of skin and marked skin. In FIG. 12, the horizontal axis represents the wavelength [nm] and the vertical axis represents absorption. As shown in FIG. 12, the absorbance 31 of marked skin is greater than the absorbance 32 of non-marked skin in the visible light wavelength band, indicating that it can easily be seen by the naked eye. Conversely, the wavelength 30 of light from the light source 2 of the phototherapy device 1000 is, for example, approximately 800 [nm], and at this wavelength, the absorbance 32 of the skin and the absorbance 31 of the marked skin exhibit equivalent values. This means that the marking does not specifically absorb light of this wavelength. Thus, even if a part of the light from the light source 2 leaks from the barrel 1 and irradiates the marking, there is no risk that the temperature of the skin on which the marking is arranged will rise, whereby treatment can safely be performed.

As described above, according to the phototherapy device according to an embodiment of the present disclosure, the light irradiation region can accurately be aligned with the treatment site.

**Claims**

1. A phototherapy device (1000) for use by a patient (400) to irradiate themselves with light at home, comprising:

   a barrel (1);
   a light source (2) which is installed inside the barrel (1) and for emitting light;
   a plurality of imaging units (3) for imaging a marking (10) placed on the patient's body at a treatment site;
   a deviation amount calculation unit (4) for calculating a deviation amount (D) between a plurality of images of the marking (10) included in a plurality of images captured by the plurality of imaging units (3);
   an image combining unit (5) for combining the plurality of images of the marking (10) based on the calculated deviation amount (D) and generating a combined image (20'; 20");
   a target position determination unit (6) for determining a position of a target (24) in the combined image (20'; 20") corresponding to an irradiation region of light from the light source; and
   a display unit (16) for overlaying and displaying to the patient (400) an image of the target (24) and the marking (10) on the combined image (20'; 20").

2. The phototherapy device (1000) according to claim 1, wherein the deviation amount calculation unit (4) is arranged for calculating the deviation amount (D) based on the center position of the marking (10) in the plurality of images of the marking (10).

3. The phototherapy device (1000) according to claim 1 or 2, wherein the plurality of imaging units (3) are arranged behind a barrel tip (13), which is a portion of the barrel (1) for contact with the patient's body, and outside of the barrel (1).

4. The phototherapy device (1000) according to any one of claims 1 to 3, wherein the marking (10) includes a first marking (11) which is larger than the target (24), and second markings (12) which are smaller than the first marking (11), and wherein the deviation amount calculation unit (4) is arranged for calculating a position of the first marking (11) based on a position of the second marking (12).

5. The phototherapy device according to any one of claims 1 to 4, further comprising a target deviation amount calculation unit (8) for calculating a target deviation amount between the target position and a position of the markings (10).

6. The phototherapy device according to any one of claims 1 to 5, further comprising an irradiation position judgment unit (7) for judging whether or not the position of the irradiation region is a predetermined irradiation region position based on whether or not the position of the marking (10) and the position of the target (24) are aligned in the combined image (20'; 20").

7. The phototherapy device according to claim 6 as dependent on claim 5, wherein the irradiation position judgment unit (7) is arranged for judging whether or not the position of the center of the irradiation region of the light from the light source (2) and the position of the center of the target (24) are aligned based on the target deviation amount.

8. The phototherapy device according to any one of claims 1 to 7, further comprising a filter which is installed in front of the plurality of imaging units (3) for filtering emitted light of the light source (2).

9. The phototherapy device according to any one of claims 1 to 8, wherein the wavelength of the emitted light of the light source (2) does not overlap with an absorption wavelength of the marking (10).

10. The phototherapy device according to any one of claims 1 to 9, wherein the light emitted from the light source (2) is infrared light with a wavelength of 600 to 12000 nm, preferably near infrared light of 780 to 2500 nm, even more preferably of 800 nm.

**Patentansprüche**

1. Phototherapievorrichtung (1000) zur Verwendung durch einen Patienten (400), um sich zu Hause mit Licht zu bestrahlen, umfassend:

   einen Zylinder (1);
   eine Lichtquelle (2), die im Inneren des Zylinders (1) installiert ist und Licht emittiert;
   eine Vielzahl von Abbildungseinheiten (3) zum Abbilden einer Markierung (10), die an einer Behandlungsstelle auf dem Körper des Patienten platziert ist;
   eine Abweichungsbetrags-Berechnungseinheit (4) zum Berechnen eines Abweichungsbetrags (D) zwischen einer Vielzahl von Bildern der Markierung (10), die in einer Vielzahl von Bildern enthalten sind, die von der Vielzahl von Abbildungseinheiten (3) aufgenommen wurden;
   eine Bildkombinationseinheit (5) zum Kombinieren der Vielzahl von Bildern der Markierung (10) basierend auf dem berechneten Abweichungsbetrag (D) und zum Erzeugen eines kombinierten Bildes (20'; 20");
   eine Zielpositions-Bestimmungseinheit (6) zum Bestimmen einer Position eines Ziels (24) in dem kombinierten Bild (20'; 20") entsprechend einem Bestrahlungsbereich des Lichts von der Lichtquelle; und
eine Anzeigeeinheit (16) zum Überlagern und Anzeigen eines Bildes des Ziels (24) und der Markierung (10) auf dem kombinierten Bild (20'; 20") für den Patienten (400).

2. Phototherapievorrichtung (1000) nach Anspruch 1, wobei die Abweichungsbetrags-Berechnungseinheit (4) dazu angepasst ist, den Abweichungsbetrag (D) basierend auf der Mittelposition der Markierung (10) in der Vielzahl von Bildern der Markierung (10) zu berechnen.

3. Phototherapievorrichtung (1000) nach Anspruch 1 oder 2, wobei die Vielzahl von Abbildungseinheiten (3) hinter einer Zylinderspitze (13), die ein Abschnitt des Zylinders (1) zum Kontakt mit dem Körper des Patienten ist, und außerhalb des Zylinders (1) angeordnet sind.

4. Phototherapievorrichtung (1000) nach Anspruch 1 bis 3, wobei die Markierung (10) eine erste Markierung (11), die größer als das Ziel (24) ist, und zweite Markierungen (12), die kleiner als die erste Markierung (11) sind, einschließt, und wobei die Abweichungsbetrags-Berechnungseinheit (4) dazu angepasst ist, eine Position der ersten Markierung (11) basierend auf einer Position der zweiten Markierung (12) zu berechnen.

5. Phototherapievorrichtung nach einem der Ansprüche 1 bis 4, weiterhin umfassend eine Zielabweichsbetrag-Berechnungseinheit (8) zum Berechnen eines Zielabweichsbetrags zwischen der Zielposition und einer Position der Markierungen (10).

6. Phototherapievorrichtung nach einem der Ansprüche 1 bis 5, weiter umfassend eine Bestrahlungspositions-Beurteilungseinheit (7) zum Beurteilen, ob die Position des Bestrahlungsbereichs eine vorbestimmte Bestrahlungsbereichsposition ist oder nicht, basierend darauf, ob die Position der Markierung (10) und die Position des Ziels (24) in dem kombinierten Bild (20'; 20") ausgerichtet sind oder nicht.

7. Phototherapievorrichtung nach Anspruch 6, abhängig von Anspruch 5, wobei die Bestrahlungspositions-Beurteilungseinheit (7) so angeordnet ist, dass sie basierend auf dem Zielabweichsbetrag beurteilt, ob die Position des Zentrums des Bestrahlungsbereichs des Lichts von der Lichtquelle (2) und die Position des Zentrums des Ziels (24) aufeinander ausgerichtet sind oder nicht.

8. Phototherapievorrichtung nach einem der Ansprüche 1 bis 7, weiter umfassend einen Filter, der vor der Vielzahl von Abbildungseinheiten (3) installiert ist, um das emittierte Licht der Lichtquelle (2) zu filtern.

9. Phototherapievorrichtung nach einem der Ansprüche 1 bis 8, wobei die Wellenlänge des von der Lichtquelle (2) emittierten Lichts sich nicht mit einer Absorptionswellenlänge der Markierung (10) überschneidet.

10. Phototherapievorrichtung nach einem der Ansprüche 1 bis 9, wobei das von der Lichtquelle (2) emittierte Licht Infrarotlicht mit einer Wellenlänge von 600 bis 12000 nm ist, bevorzugt Nahinfrarotlicht von 780 bis 2500 nm, bevorzugter von 800 nm.

**Revendications**

1. Dispositif de photothérapie (1000) pour une utilisation par un patient (400) afin qu'il s'irradie lui-même avec une lumière à domicile, comprenant :

   un barillet (1) ;
   une source de lumière (2) qui est installée à l'intérieur du barillet (1) pour émettre une lumière ;
   une pluralité d'unités d'imagerie (3) pour capturer une image d'un repère (10) placé sur le corps du patient au niveau d'un site de traitement ;
   une unité de calcul de quantité d'écart (4) pour calculer la quantité d'écart (D) entre une pluralité d'images du repère (10) faisant partie de la pluralité d'images capturées par la pluralité d'unités d'imagerie (3) ;
   une unité de combinaison d'images (5) pour combiner la pluralité d'images du repère (10) sur la base de la quantité d'écart calculée (D) et générer une image combinée (20', 20") ;
   une unité de détermination de position de cible (6) pour déterminer la position d'une cible (24) dans l'image combinée (20', 20") correspondant à une région d'irradiation de lumière depuis la source de lumière ; et
   une unité d'affichage (16) pour superposer et afficher au patient (400) une image de la cible (24) et du repère (10) sur l'image combinée (20', 20").

2. Dispositif de photothérapie (1000) selon la revendication 1, dans lequel l'unité de calcul de quantité d'écart (4) est disposée de façon à calculer la quantité d'écart (D) sur la base de la position centrale du repère (10) dans la pluralité d'images du repère (10).

3. Dispositif de photothérapie (1000) selon la revendication 1 ou 2, dans lequel la pluralité d'unités d'imagerie (3) est disposée sous une pointe de barillet (13), qui est une partie du barillet (1) destinée à venir en contact avec le corps du patient, et à l'extérieur du barillet (1).

4. Dispositif de photothérapie (1000) selon l'une quelconque des revendications 1 à 3, dans lequel le repère (10) comprend un premier repère (11) qui est plus gros que la cible (24), et des deuxièmes repères (12) qui sont plus petits que le premier repère (11), et dans lequel l'unité de calcul de quantité d'écart (4) est disposée de façon à calculer la position du premier repère (11) sur la base de la position du deuxième repère (12).

5. Dispositif de photothérapie selon l'une quelconque des revendications 1 à 4, comprenant en outre une unité de calcul de quantité d'écart de cible (8) pour calculer la quantité d'écart de cible entre la position de cible et une position des repères (10).

6. Dispositif de photothérapie selon l'une quelconque des revendications 1 à 5, comprenant en outre une unité d'estimation de position d'irradiation (7) pour estimer si oui ou non la position de la région d'irradiation est une position de région d'irradiation prédéterminée sur la base que la position du repère (10) et la position de la cible (24) sont oui ou non alignées dans l'image combinée (20', 20").

7. Dispositif de photothérapie selon la revendication 6 quand elle dépend de la revendication 5, dans lequel l'unité d'estimation de position d'irradiation (7) est disposée de façon à estimer si oui ou non la position du centre de la région d'irradiation de la lumière depuis la source de lumière (2) et la position du centre de la cible (24) sont alignées sur la base de la quantité d'écart de cible.

8. Dispositif de photothérapie selon l'une quelconque des revendications 1 à 7, comprenant en outre un filtre qui est installé en avant de la pluralité d'unités d'imagerie (3) pour filtrer la lumière émise par la source de lumière (2).

9. Dispositif de photothérapie selon l'une quelconque des revendications 1 à 8, dans lequel la longueur d'onde de la lumière émise par la source de lumière (2) ne chevauche pas la longueur d'onde d'absorption du repère (10).

10. Dispositif de photothérapie selon l'une quelconque des revendications 1 à 9, dans lequel la lumière émise par la source de lumière (2) est une lumière infrarouge ayant une longueur d'onde de 600 à 12 000 nm, de préférence une lumière proche infrarouge de 780 à 2 500 nm, mieux encore de 800 nm.

EP 4 252 835 B1

# FIG. 1

FIG. 2

EP 4 252 835 B1

FIG. 3

FIG. 4

FIG. 5

START

EXECUTE DETECTION OF FIRST MARKING — S101

S102
HAS FIRST MARKING BEEN DETECTED IN BOTH OF LEFT AND RIGHT IMAGES ?

No → 

Yes S103

CALCULATE DEVIATION AMOUNT FROM POSITIONS OF FIRST MARKING IN LEFT AND RIGHT IMAGES

S105
HAS FIRST MARKING BEEN DETECTED IN ONE OF LEFT AND RIGHT IMAGES ?

No → 

Yes S106

CALCULATE DEVIATION AMOUNT FROM RADIUS OF FIRST MARKING

EXECUTE DETECTION OF SECOND MARKINGS — S107

S108
HAVE SECOND MARKINGS BEEN DETECTED IN EITHER OF LEFT AND RIGHT IMAGES ?

No →

Yes S109

CALCULATE RADIUS OF FIRST MARKING FROM DISTANCE BETWEEN SECOND MARKINGS

CALCULATE DEVIATION AMOUNT FROM RADIUS OF FIRST MARKING — S110

USE DEVIATION AMOUNT CALCULATED FROM IMAGES ONE FRAME PRIOR — S111

COMBINE LEFT AND RIGHT IMAGES BASED ON DEVIATION AMOUNT — S104

END

EP 4 252 835 B1

# FIG. 6

20L

10L

D

20R

10R

W

# FIG. 7

20L´    21L

10L´

22

(W-D)/2

(W-D)/2

21R    10R´

20R´

20´

20L´    20R´

22

10L´    10R´

10´

# FIG. 8

20L

10L

D

W

10R

20R

FIG. 9

FIG. 10

EP 4 252 835 B1

# FIG. 11A

# FIG. 11B

EP 4 252 835 B1

# FIG. 12

22

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020004516 A
- WO 2018017811 A1
- WO 2019094808 A1